## Europäisches Patentamt

## European Patent Office

## Office européen des brevets

(19)

(11) Publication number: **0 118 483**

**B1**

(12)

# EUROPEAN PATENT SPECIFICATION

(45) Date of publication of patent specification: **25.11.87**

(21) Application number: **83902693.7**

(22) Date of filing: **25.08.83**

(86) International application number:
**PCT/HU83/00045**

(87) International publication number:
**WO 84/01037 15.03.84 Gazette 84/07**

(51) Int. Cl.⁴: **G 02 F 1/17,** G 02 F 1/13,
G 02 F 1/03, A 61 F 9/06

(54) **METHOD AND APPARATUS FOR THE OBSERVATION OF LUMINOUS PHENOMENA.**

(30) Priority: **25.08.82 HU 273582**

(43) Date of publication of application:
**19.09.84 Bulletin 84/38**

(45) Publication of the grant of the patent:
**25.11.87 Bulletin 87/48**

(84) Designated Contracting States:
**AT BE CH DE FR GB LI NL SE**

(56) References cited:
**EP-A-0 027 518
WO-A-81/02795
CH-A- 614 294
DE-A-2 553 976
DE-A-3 017 215
GB-A-1 430 183**

(73) Proprietor: **PALFI, Zoltán
Keselyü u. 1
H-1025 Budapest (HU)**

(73) Proprietor: **KREKACS, Sándor
Juhász Gy. U. 2
H-1039 Budapest (HU)**

(73) Proprietor: **FLEDRICH, István
Munkácsy Mihály u. 3/a C/16
H-2100 Gödöllö (HU)**

(73) Proprietor: **BIHARY, Sándor
DK-6690 Görding (DK)**

(72) Inventor: **PALFI, Zoltán
Keselyü u. 1
H-1025 Budapest (HU)**
Inventor: **KREKACS, Sándor
Juhász Gy. U. 2
H-1039 Budapest (HU)**
Inventor: **FLEDRICH, István
Munkácsy Mihály u. 3/a C/16
H-2100 Gödöllö (HU)**
Inventor: **BIHARY, Sándor
DK-6690 Görding (DK)**

(74) Representative: **Patentanwälte Viering &
Jentschura
Steinsdorfstrasse 6
D-8000 München 22 (DE)**

Courier Press, Leamington Spa, England.

## Description

The present invention relates to a method and a device of controlling the intensity of light passing through a light transmission varying element from a light source exhibiting intensive luminous effects.

Such intensive luminous effects are especially frequently present during heat power technological processes, i.e. welding or melting.

While observing luminous effects of said origin, the sensitivity of the human eye can adjust itself only within certain limits to the intensity of the luminous effect to be observed. This so called adaptation of the human eye is physiologically limited, and any observation with the unprotected eye of intensive light radiation leads to serious damage of the human eye.

For the observation of such luminous effects, the most comfortable and most practicable solution for an observer is an element of variable transmittance, positioned between him and the light source, the transmittance being controlled upon the intensity of the luminous effect to be observed.

Such a known device (GB—A—14 30 183) comprises liquid crystals or a depolarizer between crossed polarizers as light transmission varying element, controlled by an electric voltage, the magnitude of this voltage being a function of the intensity of the light received by a photo-electric element. As the photo-electric element is positioned behind the light transmission varying element and receives light passed through said element, an intensive luminous effect beyond the normal intensitivy of the light source will cause a reduction of the light transmittance of said varying element only after a substantial part of the intensive light has already passed the varying element.

Further, a method and device according to the pre-characterizing parts of claims 1 and 2 are known (EP—A—0027518). In this known device, however, the light transmission varying element exhibits only two light transmission conditions, the first one being a normal light transmissive condition and the second one being an eye protective, low light transmissive condition. Accordingly, if the varying element is set to the second light transmission condition, any intensive luminous effect will not cause a further reduction of the light transmittance of said varying element.

An aim of the invention is to provide a method and a device of the kind described in the pre-characterizing part of claim 1 resp. 2, by which the intensity of the light passing the light transmission varying element can be controlled to nearly constancy, the constancy depending on the preset value, thus being independent of the intensity fluctuations of the light source, and by which the light transmittance of said element can automatically be reduced just upon occurrence of an intensive luminous effect.

According to the invention this aim is reached by the features described in the characterizing part of claim 1 resp. 2. Preferred embodiments of the device are given by claims 3 to 5.

By the invention, a light source exhibiting intensive luminous effects can be observed much favourably and with higher safety from the point of view of labour safety than before, as the initial variation of light transmittance of the varying element is controlled by a signal higher than the normal control signal. In this way, the reduction of light transmittance of the varying element is fast and corresponds to the intensity of the light effect.

The features in claim 4 present the possibility of operating said element without external voltage supply whereby a lack of failure of the external supply does not affect the safety during the observation of the light source.

The invention will now be described by way of example with reference to the accompanying drawings in which:

Fig. 1 depicts the outline drawing of a device according to the invention, specified in that the light transmission varying element is a liquid crystal inserted between two polarizers,

Fig. 2 depicts the outline drawing of a device according to the invention, specified in that the light transmission varying element is composed of two variable polarizers,

Fig. 3 depicts the outline drawing of a device according to the invention specified in that at least one of the polarizers is mechanically independent of the liquid crystal, and

Fig. 4 depicts the block diagram of a light transmission control unit suitable for this purpose.

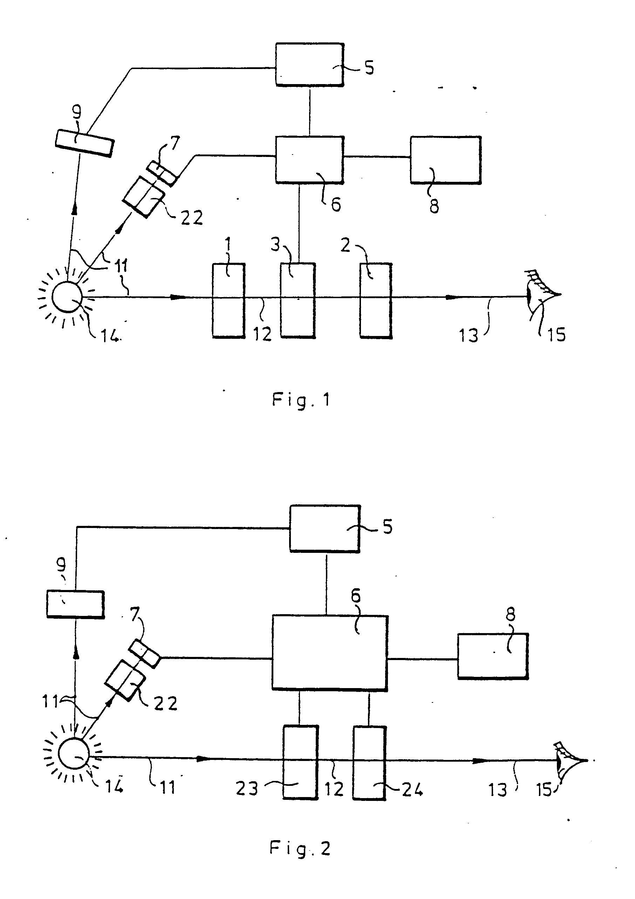

The device depicted by Fig. 1 involves a first polarizer 1 facing a light source 14, a second polarizer 2 facing an observer 15, a rotary unit 3, a transducer 5, a control unit 6, a sensing element 7, a light intensity setting means 8 and a heat or light energy utilizer 9. The light source 14 emits diffuse light beams 11 which after having passed through the first polarizer 1 are prevalant as polarized light beams 12 which, after having passed through the rotary unit 3 and the second polarizer 2, are transformed into light beams 13 suitable for inspection, while in the heat or light energy utilizer 9 and in the sensing element 7 the light beams 11 are producing electric signals or change electrical characteristics. In the surroundings of the sensing element 7 there are shields 22 inserted in.

The principal way of operation of the above cited lay-out is as follows:

The diffuse light beams 11 of the light source 14 are producing electric energy in the heat or light energy utilizer 9 which consist preferably of thermoelectric or photoelectric cells. The energy produced in this way is transduced by the transducer 5.

Sensing the intensity of the light source 14, the sensing element 7 shielded by the shields 22 gives a signal to the control unit 6 which, comparing it with the signal of the light intensity setting means 8, forms a control signal which varies the degree of rotation of the rotary unit. The shields 22 grant that the variation of the light transmission is due only to the light source 14, excluding ambiant light effects. Depending on the value given by the light intensity setting means 8, the rotary unit 3 — inserted between the second polarizer 2 and the first polarizer 1 — modifies — in accordance with the signal of the control unit 6 — the direction of polarization of light passing therethrough in such a way that the light intensity percepted by the observer 15 remains at a preset, nearly constant level.

The control unit 6 as shown in Fig. 2 is connected to a first variable polarizer 23 and to a second variable polarizer 24. The characteristic features of these first and second variable polarizers 23 and 24 are that they can vary the degree of polarization and/or the degree of rotation of the vector of the light resp. the degree of rotation of the polarizers according to the applied voltage. The way of operation of this embodiment is similar to the one depicted in Fig. 1 with the exception that the light transmission is varied by means of the variation of the input voltage of the first and the second variable polarizers 23 and 24. We have to note that it is not absolutely necessary to apply variable polarizers for both the first and the second polarizers, as a similar result can be achieved by a permanent and a variable polarizer, as well.

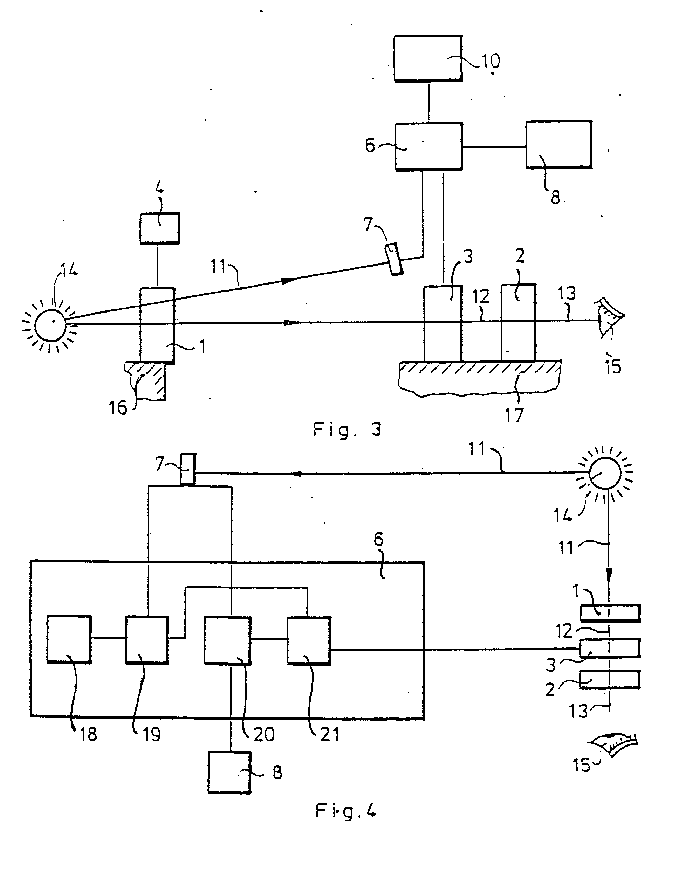

Fig. 3 shows an embodiment where the first polarizer 1 is connected to an inserting unit 4 and is arranged on a first supporting element 16. The rotary unit 3 and the second polarizer 2 are mounted on a second supporting element 17, the second supporting element 17 and the first supporting element 16 being separately movable. The control unit 6 of this embodiment is in connection with an external electrical source 10.

Fig. 4 shows the block diagram of a preferred embodiment of the control unit 6. The control unit 6 involves a start signal generator 18, e.g. an accumulator, a tripping circuit 19, a differentiator 20 and a control signal generator 21, the start signal generator 18 being connected to the tripping circuit 19, and the latter being connected to the control signal generator 21, the differentiator 20 being also connected to the control signal generator 21.

The principle of the operation of the device depicted in the block diagram of Fig. 4 is that an intensive luminous effect sensed by the sensor 7 controls the tripping circuit 19 which connects the energy stored in the accumulator 18 to the control signal generator 21 which produces a comparatively high start signal the value of which exceeding that of the normal control signal corresponding to the signal produced in the differentiator 20 in accordance with the luminous effect sensed by the sensing element 7. In this way the rotary unit 3 resp. the variable polarizer 23, 24 is "overdriven" so to say, so the required decrease of light transmission is reached in an extremely short time.

Some effective embodiments of the invention can be supplied with several sensing elements 7. Further, the sensing element 7 could be directly connected to the rotary unit 3 resp. to at least one variable polarizer.

Advantages of the device according to the invention are its relative simplicity and its safe and fast operation.

## Claims

1. Method of controlling the intensity of light passing through a light transmission varying element (3: 23, 24) from a light source (14) exhibiting intensive luminous effects, in which the light intensity of the light source (14) is directly sensed, the light transmittance of said element is controlled by a control signal in dependence upon the sensing signal and in comparison with a preset value, characterized in that at the beginning of each intensive luminous effect indicated by the sensing signal a start signal acting on the light transmission varying element is triggered which start signal is different from and higher than the normal control signal.

2. Device for controlling the light intensity passing through a light transmission varying element (3; 23; 24) from a light source (14) exhibiting intensive luminous effects, comprising a sensing element (7) positioned so that it directly senses the light intensity of the light source (14), a power source and a control unit (6) connected to the sensing element (7) and equipped with a control signal generator (21) connected to the light transmission varying element (3; 23; 24) so that it controls the transmittance in comparison with a preset value, characterized in that the control unit (6) comprises an adjustable setting means (8) for determining the light intensity to be transmitted through the transmission varying element (3; 23; 24) and a start signal generator (18) comprising a tripping circuit (19) connected to the sensing element (7) and the control signal generator (21) to make the latter supply to the light transmission varying element a start signal higher than the normal control signal, at the beginning of each intensive luminous effect sensed by the sensing element (7).

3. Device according to claim 2, characterized in that the start signal generator comprises an accumulator (18).

4. Device according to claims 2 or 3, characterized in that the power source is a heat or light energy utilizer (9) driven by the heat or light energy of the light source (14).

5. Device according to one of claims 2 to 4, characterized in that the sensing element (7) is connected to a RC-network.

## Patentansprüche

1. Verfahren zum Steuern der Intensität von Licht, das von einer intensiv strahlenden Lichtquelle (14) durch ein transmissionsverändernes Element (3, 23, 24) fällt, wobei die Intentsität der Lichtquelle (14) direkt gemessen wird und die Transmittanz des Elements von einem Steuersignal in Abhängigkeit von einem Meßsignal und im Vergleich mit einem vorgegebenen Wert gesteuert wird, dadurch gekennzeichnet, daß zu Beginn jeder intensiven Strahlung, die von dem Meßsignal aufgezeigt wird, ein Startsignal ausgelöst wird, das auf ein transmissionsveränderndes Element einwirkt, wobei dieses Startsignal von dem normalen Steuersignal verschieden und höher als dieses ist.

2. Vorrichtung zum Steuern der Intensität von Licht einer intensiv strahlenden Lichtquelle (14), das durch ein transmissionsveränderndes Element (3, 23, 24) fällt, mit einem Meßelement (7), das so positioniert ist, daß es die Intensität der Lichtquelle (14) direkt mißt, einer Energiequelle une einer Steuereinheit (6), die mit dem Meßelement (7) verbunden ist und mit einem Steuersignalgenerator ausgestattet ist, der mit dem transmissionsverändernden Element (3, 23, 24) verbunden ist, so daß es die Transmittanz im Vergleich mit einem vorgegebenen Wert steuert, dadurch gekennzeichnet, daß die Steuereinheit (6) eine justierbare Einstelleinrichtung (8) zum Bestimmen der Intensität des durch das transmissionsverändernde Element (3, 23, 24) durchgelassenen Lichtes sowie einen Startsignalgenerator (18) mit einer mit dem Meßelement (7) verbundenen Auslöseschaltung (19) und einen Steuersignalgenerator (21) enthält, der bewirkt, daß die Auslöseschaltung (19) das transmissionsverändernde Element mit einem Startsignal versorgt, das zu Beginn jeder vom Meßelement (7) gemessenen intensiven Strahlung höher als das normale Steuersignal ist.

3. Vorrichtung nach Anspruch 2, dadurch gekennzeichnet, daß der Startsignalgenerator einen Akkumulator (18) enthält.

4. Vorrichtung nach den Ansprüchen 2 und 3, dadurch gekennzeichnet, daß die Energiequelle ein Hitze- oder Lichtenergiewandler (9) ist, der von der Hitze- oder der Lichtquelle (14) gespeist wird.

5. Vorrichtung nach einem der Ansprüche 2 bis 4, dadurch gekennzeichnet daß das Meßelement (7) an ein RC-Glied angeschlossen ist.

## Revendications

1. Procédé de commande de l'intensité lumineuse traversant un élément variable de transmission de lumière (3, 23, 24) et provenant d'une source lumineuse (14) présentant des effets lumineux intenses, dans lequel on capte directement l'intensité lumineuse de la source lumineuse (14), on commande la transmittance lumineuse du dit élément au moyen d'un signal de commande fonction du signal capté et comparé avec une valeur prédéterminée, caractérisé en ce que, au commencement de chaque effet lumineux intense indiqué par le signal capté, on déclenche un signal de départ agissant sur l'élément variable de transmission de lumière, ce signal de départ étant différent du signal de commande normal et supérieur à celui-ci.

2. Dispositif de commande de l'intensité lumineuse traversant un élément variable de transmission de lumière (3, 23, 24) provenant d'une source lumineuse (14) présentant des effets lumineux intenses, comprenant un capteur (7) disposé de telle manière qu'il capte directement l'intensité lumineuse de la source lumineuse (14), une source de puissance et une unité de commande (6) reliée au capteur (7) et équipée d'un générateur de signal de commande (21) connecté à l'élément variable de transmission de lumière (3, 23, 24), de manière qu'il commande la transmittance en comparaison avec une valeur prédéterminée, caractérisé en ce que l'unité de commande (6) comprend des moyens de réglage ajustables (8) pour fixer l'intensité lumineuse à transmettre à travers l'élément variable de transmission (3, 23, 24) et un générateur de signal de départ (18) comprenant un circuit de déclenchement (19) connecté au capteur (7) et au générateur de signal de commande (21) pour que ce dernier fournisse à l'élément variable de transmission de lumière, un signal de départ supérieur au signal de commande normal, au commencement de chaque effet lumineux intense capté par le capteur (7).

3. Dispositif selon la revendication 2, caractérisé en ce que le générateur du signal de départ comprend un accumulateur (18).

4. Dispositif selon les revendications 2 ou 3, caractérisé en ce que la source de puissance est un élément (9) utilisant l'énergie thermique ou lumineuse et actionné par l'énergie thermique ou lumineuse de la source de lumière (14).

5. Dispositif selon l'une des revendications 2 à 4, caractérisé en ce que le capteur (7) est relié à un circuit-RC.

Fig. 1

Fig. 2

Fig. 3

Fig. 4